(19) Europäisches Patentamt
European Patent Office
Office européen des brevets

(11) Publication number: **0 249 349 B1**

(12) **EUROPEAN PATENT SPECIFICATION**

(45) Date of publication of patent specification: **14.10.92**

(51) Int. Cl.[5]: **C07D 207/16**, A61K 31/40

(21) Application number: **87304452.3**

(22) Date of filing: **19.05.87**

(54) **Difluoroketo compounds.**

(30) Priority: **05.06.86 GB 8613704**
**16.01.87 US 3993**

(43) Date of publication of application:
**16.12.87 Bulletin 87/51**

(45) Publication of the grant of the patent:
**14.10.92 Bulletin 92/42**

(84) Designated Contracting States:
**BE CH DE ES FR GB IT LI NL**

(56) References cited:
**EP-A- 0 124 317**
**EP-A- 0 130 679**
**EP-A- 0 204 571**

**BIOCHEMISTRY, vol. 24, no. 8, April 9, 1985, MICHEL H. GELB et al. "Fluoro Ketone Inhibitors of Hydrolytic Enzymes" pages 1813-1817**

**JOURNAL OF MEDICINAL CHEMISTRY, vol. 28, no. 11, November 1985, SUVIT THAISRIVONGS et al. "Disfluorostatine- and Difluorostanone- Containing Peptides as Potent and Specific Renin Inhibitors"**

**TETRAHEDRON LETTERS, vol. 27, no. 2, 1986; BARBARA IMPERIALI et al. "A Versatile Synthesis of Peptidyl Fluoromethyl Ketones", pages 135-138**

(73) Proprietor: **ICI AMERICAS INC.**
**Concord Pike & New Murphy Road**
**Wilmington Delaware 19897(US)**

(72) Inventor: **Trainor, Amy Diane**
**7 Marshall Road**
**Glenmills, PA 19342(US)**
Inventor: **Stein, Mark Morris**
**1114 Grinnell Road**
**Wilmington DE, 19803(US)**

(74) Representative: **Smith, Stephen Collyer et al**
**ICI Group Patents Services Dept. PO Box 6**
**Shire Park Bessemer Road**
**Welwyn Garden City Herts, AL7 1HD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

### Background and Summary of the Invention

The present invention relates to certain difluoro keto compounds which are human leukocyte elastase (HLE) inhibitors and are also useful as research tools in pharmacological and related studies and in the treatment of tissue degenerative diseases such as pulmonary emphysema, atherosclerosis, rheumatoid arthritis and osteoarthritis in warm blooded animals. The invention also includes intermediates useful in the synthesis of these peptide derivatives, processes for preparing them, pharmaceutical compositions containing such peptide derivatives and methods for their use. Proline based peptide aldehydes are disclosed in European Patent Application 84302621.2. Fluoroketone inhibitors of hydrolytic enzymes are disclosed in Gelb, M.H. et al, Biochemistry (1985) 24, 1813-1817 for non-serine proteases. Imperiali, B. et al, Tetra. Letters (1986) 27, No. 2, 135-138 shows selected fluoromethyl ketones. Thaisrivongs, S. et al, J. Med. Chem. (1985) 28, No. 11, 1553-1555 discloses selected fluoro ketones as renin inhibitors.

European patent application, publication no. 130679 discloses certain irreversible inhibitors of a range of cysteine and serine proteases. European patent application, publication no. 204571 discloses certain structurally related difluoroketone inhibitors of human leukocyte elastase.

### Description of the Invention

The difluoro keto compounds of the present invention may be represented by the following formula Ib:

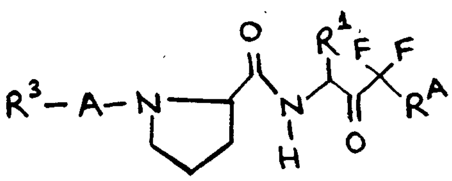

Ib

wherein $R^1$ is (1-3C) alkyl; $R^3$ is benzyl;
$R^A$ is a group of the formula -CO.X.$R^B$ wherein X.$R^B$ is methyl or X is the group -NH- and $R^B$ is selected from:-
$-CH_2CH_2CO_2CH_2CH_3$, $-CH_2CH_2$(2-pyridyl), $-CH_2CH_2OH$, $-CH_2CH_2CH_2OH$, $-CH_2CH(OH)$(phenyl), $-CH_2CH_2CO_2H$ and $-CH_2CH_2CONHS(O)_2$(4-chlorophenyl); and A is oxycarbonyl; or a pharmaceutically acceptable salt thereof.

More particular values for compounds of the invention include those having the following members of the groups defined above:

$R^1$ is an alkyl group containing 3 carbons, especially isopropyl;

The salts of the compounds of formula Ib include pharmaceutically acceptable base or acid addition salts such as those made with a mineral acid, e.g., hydrochloric, or an organic acid such as citric, maleic, fumaric or acetic. Base-addition salts include those made with alkali metal hydroxides such as sodium hydroxide, alkali metal carbonates and bicarbonates, alkaline earth hydroxides and organic amine salts. Such salts may be prepared by dissolving the peptide derivative in a mixture of water and a water-miscible organic solvent, adding an aqueous solution of the base and recovering the salt from the aqueous solution.

The preferred compounds of the present invention are of the S configuration (i.e., that of the naturally occurring L-amino acids) at chiral center identified by * in formulae IIIb below and the methods of synthesis described below provide isomers with the S configuration at the chiral center identified by symbol # or isomeric mixtures as a result of the R and S configurations at the chiral center identified by the symbol #. It is generally preferred that the compounds have the S configuration at the center identified by the symbol #.

(Formula set out on pages following Examples) IIIb

As will be appreciated by those skilled in the art, the activity of the individual isomers is not the same, and it is therefore preferred to utilize the more active isomer. The present invention includes compounds resulting from the S and/or R configuration at the chiral center labelled #.

$R^B$ may have a chiral center. The present invention includes compounds of formula Ib wherein the chiral center included in $R^B$ is of the S or R configuration.

As will be appreciated by those skilled in the art, the difluoro ketone derivatives can exist as solvates, particularly hydrates, formula IVb, and these are encompassed by the present invention.

(Formula set out on pages following Examples) IVb

It is preferred to prepare the difluoro keto compounds of the present invention from commercially available alpha amino acids (i.e., those in which the $NH_2$ group is attached to the carbon atom next to the -COOH group).

According to a further feature of the invention there are provided pharmaceutical compositions comprising a pharmaceutically effective amount of at least one peptide derivative of formula Ib and a pharmaceutically acceptable diluent or carrier.

The difluoro keto compounds of formula Ib may be prepared as follows.

Method A

A compound of formula Ib may be prepared from the corresponding alcohol of formula Vb:

(Formula set out on pages following Examples) Vb

by an oxidative process. Methods which are useful include the use of oxalyl chloride, DMSO and a tertiary amine (see Marx, M., et al., J. Org. Chem., (1984) 49, 788-793, with the best results being obtained with 10-20 equivalents of oxidizing agent), the use of acetic anhydride and DMSO, the use of chromium trioxide pyridine complex in methylene chloride, and the use of Dess-Martin periodinane [1,1,1-triacetoxy-2,-1-benzoxiodol-3(3H)-one] (method of Dess, D. B. et al., J. Org. Chem., (1983) 48, 4155-56).

The alcohols of formula Vb may be prepared as follows:

(i) When $R^A$ is formula II with X = NH, an aldehyde of the following formula VIb:

(Formula set out on pages following Examples) VIb

may be reacted with ethyl 2-bromo-2,2-difluoroacetate (obtained from SCM, Specialty Chemicals) and Zn in refluxing THF (see Hallinan, E.A., et al., Tetrahedron Letters, (1984) 25 (#22), 2301-2302) to give a compound of the following formula VIIb:

(Formula set out on pages following Examples) VIIb

which in turn may be reacted with an amine of formula Vd:

(Formula set out on pages following Examples) Vd

in ethanol to give the corresponding alcohol of formula VIIIb:

(Formula set out on pages following Examples) VIIIb

Compounds of VIIIb are selected compounds of formula Vb where $R^A$ is formula II with X = NH.

The aldehydes of formula VIb may be prepared by oxidation of the corresponding alcohol of formula XIVb:

(Formula set out on pages following Examples) XIVb

(e.g., oxidation conditions as described in M. Marx, et al., J. Org. Chem., (1984) 49, 788-793), or by hydrolysis or transacetalization of the corresponding acetal of formula XVb:

(Formula set out on pages following Examples) XVb

See, for example, the preparation of such compounds as described in European Patent Application 84302621.2.

Compounds of formula XIVb (for use in making compounds of formula VIb) may be prepared by reacting an amino alcohol of formula XVI:

(Formula set out on pages following Examples) XVI

with an appropriate free acid of formula XVIIb:

(Formula set out on pages following Examples) XVIIb

by standard peptide coupling procedures using methods commonly known to those skilled in the art, such as those described in M. Bodanszky, Principles of Peptide Synthesis, Springer-Verlag, Berlin, (1984), and The Peptides. Analysis, Synthesis and Biology (ed. E. Gross and J. Meinhofer), Vols. 1-5, (Academic Press, New York) 1979-1983. A compound of formula XVIIb may be prepared by standard peptide coupling and deprotection procedures as described above. The amino alcohols of formula XVI (when not commercially available) may be prepared from the corresponding alpha-amino acids of the formula $H_2NCHR^1COOH$ by reaction with a reducing agent such as diborane. See U.S. Patent 3,935,280 to Lane.

Similarly, a compound of formula XVb may be prepared by reacting an amino acetal of formula XVIIIa:

(Formula set out on pages following Examples) XVIIIa

with an appropriate acid of formula XVIIb or by standard peptide coupling procedures. The amino acetal of formula XVIIIa can be prepared as described in Examples (1a-1d).

An acetal of formula XVb may be prepared, when appropriate, by an acid catalyzed acetalization of a compound of formula VIb (e.g., with triethylorthoformate in absolute ethanol acidified with p-toluenesulfonic acid at room temperature).

An alternate method for the preparation of a compound of formula VIIIb from a compound of formula VIIb comprises reacting a compound of formula VIIb with about 1.25 equivalents of 1N NaOH in $CH_3OH$ to give compounds of formula IXb:

(Formula set out on pages following Examples) IXb

followed by reaction of the compound of formula IXb with a compound of formula Vd Using HOBT, WSCDI and THF.

Amines of formula Vd are generally commercially available or may be made by standard techniques of organic chemistry and by analogy with the synthesis of known, structurally similar compounds.

(ii) when, taken together, $XR^B$ is $CH_3$, a compound of formula Vb may be prepared by reacting a corresponding compound of formula XIIb with a Grignard reagent of formula $CH_3MgBr$.

## Methods B and C

A compound of formula Ib where $R^A$ is formula II with $X = NH$ may be prepared from the corresponding ester, such as the ethyl ester of formula XXVIb:

(Formula set out on pages following Examples) XXVIb

by reacting the ester with an appropriate amine of formula Vd in an appropriate solvent such as ethanol to afford the corresponding compound of formula Ib or, alternatively, from the corresponding acid of formula XXVIIb:

(Formula set out on pages following Examples) XXVIIb

by coupling the acid with an amine of formula Vd (using similar conditions to those described above for the conversion of IXb to VIIIb) to afford the corresponding compound of formula Ib. A starting ester of formula XXVIb may be obtained, for example, by oxidation of the corresponding alcohol of formula VIIb using one of the oxidation methods described in Method A. A starting acid of formula XXVIIb may be obtained, for example, by hydrolyzing the corresponding ester of formula XXVIb using a similar method to that described above for the hydrolysis of a compound of formula VIIb to a compound of formula IXb.

## Method D

A compound of formula Ib where $R^B$ contains a carboxy group may be prepared by decomposing the ester group of a corresponding compound of formula Ib where $R^B$ contains an ester group.

It will be appreciated that the decomposition can be performed using any one of a variety of procedures well known in the art of organic chemistry. Thus, it may be carried out, for example, by conventional hydrolysis under acid or base conditions, adjusted as necessary to minimize any hydrolytic removal of other functional groups in the molecule.

A preferred method for decomposing an ester of formula Ib comprises reacting the ester with a suitable base, for example, an alkali or alkaline earth metal hydroxide or carbonate (such as lithium hydroxide, potassium hydroxide, sodium hydroxide, calcium hydroxide or potassium carbonate) in a suitable, aqueous solvent or diluent, for example, water, optionally together with a water-miscible organic cosolvent, such as methanol, and conveniently at or near ambient temperature. When such a method is employed, the resultant carboxylic acid of formula Ib where $R^B$ contains a carboxy group is initially obtained as the corresponding salt of the base used for the hydrolysis and may be isolated as such or converted to the free acid form by a conventional acidification procedure.

## Method E

A compound of formula Ib where $R^B$ contains an acylsulfonamide group of the sulfonamidocarbonyl type may be prepared by reacting the corresponding compound of formula Ib where $R^B$ contains a carboxy group with an appropriate sulfonamide in the presence of a dehydrating agent, for example, with 1-ethyl-3-(3-dimethylaminopropyl)carbodiimide, or with a hydrochloride or hydrobromide salt thereof optionally together with an organic base, for example, 4-(dimethylamino)pyridine, in the presence of a suitable solvent or diluent, for example, dichloromethane, at a temperature in the range of, for example, 10 to 50 °C, but preferably at or near ambient temperature.

As will be obvious to one skilled in the art, it may be desired to convert a compound of formula Ib into another compound of formula Ib using a standard method well known in the art.

Also, it may be desired to optionally use a protecting group during all or portions of the above described processes; the protecting group may then be removed when the final compound is to be formed.

(See Greene, T.W., Protective Groups in Organic Synthesis, Wiley-Interscience, New York (1981).)

When a pharmaceutically acceptable salt is desired or required, it may be obtained using standard procedures well known in the art, for example, by further reacting a suitably acidic compound of formula Ib with a suitable base affording a physiologically acceptable cation or by further reacting a sufficiently basic compound of formula Ib with a suitable acid affording a physiologically acceptable anion.

Inhibition Measurements:

The ability of compounds of the invention to act as elastase inhibitors may be initially determined by the ability of a compound of the invention to inhibit the action of human leukocyte elastase (HLE) on a low molecular weight peptide substrate. The potency of an inhibitor is evaluated by obtaining a kinetic determination of the dissociation constant, $K_i$, of the complex formed from the interaction of the inhibitor with HLE. The substrate used was the anilide methoxysuccinyl-L-alanyl-L-alanyl-L-prolyl-L-valine-p-nitroanilide as described by K. Nakajima et al. in J. Biol. Chem., (1979) 254, 4027-4032 and by T. Teshima et al. in J. Biol. Chem., (1982) 257, 9, 5085-5091. The HLE enzyme used in these studies may be obtained from Elastin Products of St. Louis, Missouri or can be purified according to B. R. Viscarello et al. in Preparative Biochemistry, (1983) 13, 57-67 as follows, all work being done in a cold room at 4 ° C.

Salt Extraction-DNase Treatment: The starting material, 193 g of purulent sputum, was homogenized with 200 ml of cold distilled water and centrifuged at 30,000 x gravity for 20 min. at 4 ° C. The supernatant was discarded and the pellet extracted with high salt and treated with DNase as per the method of D. Y. Twumasi et al. in J. Biol. Chem., (1977) 252, 1917-1926. Chromatography on Elastin Agarose: The precipitate from the DNase digest was taken up in two 40 ml portions of 50 mM Tris, 1.0 M NaCl, pH 8; the suspension was centrifuged and the resulting supernatant applied directly to a column of soluble elastin-Sepharose 4B (2.5 x 20 cm). The column was washed with equilibrating buffer (50 mM Tris, 50 mM NaCl, pH 8.0) until the optical density at 280 nm ($OD_{280}$) of the eluate returned to baseline. Additional contaminating protein was eluted with two column volumes of 50 mM acetate, 1.0 M NaCl, pH 5.0. Elastase and cathepsin G (HLC-G) were finally eluted with 50 mM acetate, 1.0 M NaCl, 20% DMSO, pH 5.0. The column was developed at 6 ml/min with the collection of 10 ml fractions. The active fractions were pooled, dialyzed vs. two 6 liter changes of 50 mM acetate, 0.1 M NaCl, pH 5.5, and concentrated to 40 ml on an Amicon® ultrafiltration unit (YM-10 membrane). CM-Chromatography: The concentrated active fraction was applied to a column of CM-Sephadex® C-50 (2.2 x 10 cm) previously equilibrated with 50 mM acetate, 0.1 M NaCl, pH 5.5 and the column then washed with this buffer to remove contaminating protein. Elution was continued with 50 mM acetate, 0.2 M NaCl, pH 5.5 and resulted in the displacement of a peak of activity assayed against Bz-L-Phe-L-Val-L-Arg-pNA. HLE was next eluted with the acetate buffer containing 0.45 M NaCl, while elution of HLC-G required the presence of 1.0 M NaCl in the buffer as described by R. Martodam et al. in Preparative Biochemistry, (1979) 9, 15-31. This column was developed at 30 ml/hr with the collection of 5.5 ml fractions. From the thus purified HLE, a standard rate of production of p-nitroaniline was measured at 25 ° C spectrophotometrically in the visible spectrum at 410 nanometers with automatic data acquisition from a Cary 210 spectrophotometer obtained from Varian Associates. Reactions were initiated by injection of 10 microliters of the HLE solution into a 3 milliliter cuvette containing 2.89 milliliters of buffer (10 millimolar sodium phosphate, 500 millimolar NaCl, pH 7.6), 50 microliters substrate solution in DMSO, and 50 microliters of DMSO. Initial, steady-state reaction velocities of p-nitroaniline production were calculated by a fit of the experimental data to a linear dependence on time by linear least squares. This velocity, determined with no inhibitor present, was used as a standard in the calculation of inhibitor $K_i$ values.

As a general rule, the peptide derivatives of the present invention were found to be "slow-binding" inhibitors of HLE and therefore required special methods of analysis to accurately determine $K_i$ values for their inhibition of HLE (see Williams, J. W. and Morrison, J. F., Meth. Enz. (1979) 63, 437 for a description of these methods.) In a typical experiment, 2.89 ml of buffer (10 millimolar sodium phosphate, 500 millimolar sodium chloride, pH 7.6), 50 microliters of inhibitor solution in DMSO, and 50 microliters of substrate solution in DMSO were added to a 3 milliliter cuvette. The cuvette was stoppered, inverted several times to mix its contents and maintained at (25 ° C) in the spectrophotometer. After a period of five minutes to allow the reaction solution to come to thermal equilibrium, 10 microliters of stock enzyme solution were added to the cuvette to initiate the reaction. Duplicate or triplicate runs were done at zero inhibitor concentration and at least three non-zero inhibitor concentrations. $K_i$ values were calculated according to methods outlined in the above reference by Williams and Morrison. The $K_i$ values for selected compounds were less than $10^{-7}$.

Animal Models

Animal models of emphysema include intratracheal (i.t.) administration of an elastolytic protease to cause a slowly progressive, destructive lesion of the lung. These lesions are normally evaluated a few weeks to a few months after the initial insult. However, these proteases also induce a lesion that is evident in the first few hours. The early lesion is first hemorrhagic, progresses to an inflammatory lesion by the end of the first 24 hours and resolves in the first week post insult. To take advantage of this early lesion, the following model was used.

Hamsters are first lightly anesthetized with methohexital sodium (Brevital® from Eli Lilly). Phosphate buffered saline (PBS) pH 7.4, either alone or containing 400 µg of human leukocyte elastase (HLE), is then administered directly into the trachea. Twenty-four hours later the animals are killed and the lungs removed and carefully trimmed of extraneous tissue. Following determination of wet lung weight, the lungs are lavaged with PBS and total lavaegable red and white cells recovered are determined. The values for wet lung weights, total lavageable red cells and total lavageable white cells are elevated in a dose-dependent manner following administration of HLE. Compounds that are effective elastase inhibitors can prevent or diminish the severity of the enzyme-induced lesion resulting in lower wet lung weight and reduced values for total lavageable cells, both red and white, relative to administration of HLE alone. Compounds can be evaluated by administering them either with or at various times prior to administration of HLE to determine their utility in preventing an HLE lesion. Compounds of this invention produced statistically significant reductions in wet lung weight and total lavageable cells relative to HLE alone.

Compounds of the present invention exhibited activity in at least one of the tests described above under Inhibition Measurement or Animal Model. It should be noted that there was not always a direct correlation between the activities of the compounds measured as $K_i$ values in the Inhibition Measurement test and the reduced values for total lavageable cells and wet lung weights relative to the administration of HLE alone obtained in the Animal Model test. It is thought that the Animal Model test is more predictive of the activity of such compounds in the treatment of emphysema.

Pharmacokinetics: Male Syrian hamsters (80 to 120g) are injected intravenously with the test compound. Prior to injection and at varying time periods thereafter, they are lightly anesthetized with ether and blood samples of approximately 0.2 ml each are withdrawn by cardiac puncture. The blood is expressed into 2 ml centrifuge tubes and allowed to clot for one hour. The sample is then centrifuged and the serum removed.

Drug levels are determined by first inactivating endogenous elastase inhibitors by incubation of 50 microliters of serum with an equal volume of buffer containing 5 mg/ml bovine pancreatic trypsin for 5 min. The trypsin inactivated serum (10 microliters) is then added to a 0.52 ml cuvette containing buffer made 20 nM with respect to HLE. After an additional 30 min. incubation, the reaction is started by the addition of substrate (350 microliters) (MeOSuc-L-Ala-L-Ala-L-Pro-L-Val-pNA, 1.6 mM) and the reaction monitored spectrophotometrically at a wavelength of 410 nM. For comparative purposes, serum persistence of the test compounds is determined in the following manner:

Percent inhibition of serum samples was calculated as follows:

$$\text{percent inhibition} = \frac{Vo - Vi}{Vo} \times 100$$

The compounds of the present invention may be administered to a warm-blooded animal in need thereof, particularly a human, for the treatment of conditions of pulmonary emphysema, atherosclerosis, rheumatoid arthritis, and osteo arthritis, but in particular for emphysema. The mode of administration may be oral, parenteral, including the subcutaneous deposit by means of an osmotic pump, or via a powdered or liquid aerosol. These may be conventionally formulated in an oral or parenteral dosage form by compounding about 10 to 250 mg per unit of dosage with conventional vehicle, excipient, binder, preservative, stabilizer, flavor or the like as called for by accepted pharmaceutical practice e.g. as described in U.S. Patent No. 3,755,340. For parenteral administration, a 1 to 10 ml intravenous, intramusular or subcutaneous injection would be given containing about 0.02 to 10 mg/kg of body weight of a compound of the invention 3 or 4 times daily. The injection would contain a compound of the invention in an aqueous isotonic sterile solution or suspension optionally with a preservative such as phenol or a solubilizing agent such as ethylenediaminetetraacetic acid (EDTA). In a powdered aerosol, compounds of the invention may be administered in the same manner as cromolyn sodium via a Spinhaler® turbo-inhaler device obtained from Fisons Corp. of Bedford, Massachusetts at a rate of about 0.1 to 50 mg per capsule, 1 to 8 capsules being

administered daily for an average human. Each capsule to be used in the Spinhaler® contains the required amount of a compound of the invention with the remainder of the 20 mg capsule being a pharmaceutically-acceptable carrier such as lactose. In a liquid aerosol, the compounds of the invention are administered at the rate of about 100 to 1000 micrograms per "puff" or activated release of a standard volume of propellant. The liquid aerosol would be given at the rate of 1 to 8 puffs per day with variation in dosages due to the severity of the condition being treated, the weight of the patient and the particle size distribution of the aerosol since smaller particles will achieve greater lung penetration. Propellants, e.g., a fluorinated hydrocarbon or isobutane, containers, valves and actuators for liquid aerosols are described by L. Lachman et al. in "The Theory and Practice of Industrial Pharmacy", Lea and Febiger, Philadelphia (1976).

In the following Examples and throughout the specification, the following abbreviations are used: atm (atmospheres) with $1.013 \times 10^5$ Pascals = 1 atm; bp (boiling point); ° C (degrees Centigrade); g (grams); hr (hours); mg (milligrams); min (minutes); ml (milliliters); mmol (millimoles); mp (melting point); N (normal); nm (nanometers); nM (nanomolar); $R_f$ (relative mobility in TLC); TLC (thin layer chromatography); DCC (dicyclohexylcarbodiimide); DMF (dimethylformamide); DMSO (dimethyl sulfoxide); $Et_2O$ (diethyl ether); EtOAc (ethyl acetate); HOAc (acetic acid); WSCDI (water soluble carbodiimide; 1-ethyl-3-(3-dimethylaminopropyl)carbodiimide hydrochloride); Bz (benzoyl); HOBT (1-hydroxybenzotriazole); MeOH (methyl alcohol); Pd/C (palladium on charcoal catalyst); pNA (paranitroanilide); DMAP (4-dimethylaminopyridine); Ø (phenyl group); NMM (N-methylmorpholine); THF (tetrahydrofuran); CBZ (benzyloxycarbonyl); t-BOC (tertiarybutyloxycarbonyl); $t_R$ (HPLC retention time in min); HPLC (high performance liquid chromatography); TEA (triethylamine); TFA (trifluoroacetic acid); $Ac_2O$ (acetic anhydride); RT (room temperature); e.g. (for example); supra (above); DAST (diethylaminosulfurtrifluoride); vs. (versus); Dibal (diisobutylaluminum hydride); Zorbax® ODS analytical column (4.6 mm x 25 cm) also called "$C_{18}$ column"; and Phenomenex Zorbax $C_8$ (25cm x 4.6mm) also called "$C_8$ column". In addition, C, H, N, etc. (the conventional symbols for the elements) are used, and conventional abbreviations for amino acids, e.g. proline (Pro), valine (Val) etc. are also used. It is to be understood that generic terms such as "(1-3C)alkyl" include both straight and branched chain alkyl radicals, but references to individual alkyl radicals such as "propyl" include only the straight chain ("normal") radical, branched chain isomers such as "isopropyl" being specifically referred to. $^1H$ NMR data is given for values of delta using tetramethylsilane as an internal standard. Where needed, multiple runs of selected processes were done to obtain additional product.

Flash chromatography was carried out on Merck Kieselgel (Art 9385) and column chromatography on Merck Kieselgel 60 (Art 7734) [these materials were obtained from E. Merck, Darmstadt, W. Germany]; or flash and column chromatographies were done on acidic silica gel (J. T. Baker Chemical Co., low pH, Number 7290-R); thin layer chromatography (TLC) was carried out on Analtech 0.25 mm silica gel GHLF plates (Art 21521), obtainable from Analtech, Newark, De, USA;

No overt toxicity has been observed for these compounds.

## Example 1

2-[[[4-[(3-Ethoxy-3-oxopropyl)amino]-3,3-difluoro-1-(1-methylethyl)-2,4-dioxobutyl]amino]carbonyl]-1-pyrrolidinecarboxylic acid phenylmethyl ester (Formula Ib, $R^A$ = Formula II, $R^1 = CH(CH_3)_2$, $R^3A$ = $ØCH_2OCO$-, $X = NR^C$, $R^B = CH_2CH_2COOCH_2CH_3$, $R^C = H$)

a. N-Benzyloxycarbonyl-L-valinol (Formula XIVa, $R^3A$ = $ØCH_2OCO$-, $R^1 = CH(CH_3)_2$)

Benzyl chloroformate (91.0 g, 0.532 mol, 95% purity) was added dropwise over a period of 1 hr to a pre-cooled (0 ° C) solution of L-valinol (50.0 g, 0.484 mol) and triethylamine (60.0 g, 0.6 mol) in $CHCl_3$ (1500 ml). The reaction mixture was stirred for 1 hr at 0 ° C and then allowed to warm to room temperature over 2 hr. The reaction mixture was concentrated under vacuum. EtOAc (1500 ml) was added to the resulting residue and the organic solution was washed with aqueous 1N NaOH and brine. The organic phase was dried over $MgSO_4$, filtered and concentrated under vacuum. The resulting residue was purified by flash chromatography on a column of silica gel (6cm x 30cm) using a stepwise gradient of $Et_2O$:hexane (1:5) followed by pure $Et_2O$ to give the product (91.4 g) as a white waxy solid; TLC, $R_f$ = 0.23, silica gel, hexane:$Et_2O$ (50:50).

b. N-Benzyloxycarbonyl-L-valinal (Formula VIa, $R^3A$ = $ØCH_2OCO$-, $R^1 = CH(CH_3)_2$)

A solution of DMSO (107.2 g, 1.372 mol) in $CH_2Cl_2$ (150 ml) was added dropwise over 0.5 hr to a pre-cooled (-60 ° C), stirred solution of oxalyl chloride (87.1 g, 0.686 mol) in $CH_2Cl_2$ (800 ml) under a nitrogen

atmosphere. The temperature of the mixture rose to -45°C. The reaction mixture was then warmed to -30°C. A solution of the product of Example 1a (81.5 g, 0.343 mol) in $CH_2Cl_2$ (300 ml) was added dropwise over 45 min at -30°C. The reaction mixture was stirred for 50 min at -25°C, cooled to -40°C and a solution of diisopropylethyl amine (177.4 g, 1.372 mol) in $CH_2Cl_2$ (250 ml) was added dropwise over 45 minutes at -40°C. The reaction mixture was stirred for 1 hr as it warmed to room temperature. The reaction mixture was diluted with $CH_2Cl_2$ (1500 ml) and the organic phase was washed with aqueous 1N HCl and then concentrated under vacuum to give the product (98 g) as a green oil which was used immediately without further purification; TLC, $R_f = 0.48$, silica gel, hexane:$Et_2O$ (50:50).

c. N-Benzyloxycarbonyl-L-valinal diethylacetal (Formula XVa, $R^3A = \emptyset CH_2OCO$-, $R^1 = CH(CH_3)_2$)

Triethyl orthoformate (700 g, 4.723 mol), absolute EtOH (800 ml) and p-toluenesulfonic acid monohydrate (5.0 g, 0.026 mol) were added to the product of Example 1b (81 g, 0.343 mol). The mixture was stirred for 10 minutes at room temperature and then concentrated under vacuum. The resulting residue was dissolved in $Et_2O$ and washed with saturated aqueous $NaHCO_3$. The organic phase was dried over $Na_2SO_4$, filtered and concentrated under vacuum to give a crude product. This product was purified by flash chromatography with silica gel using a stepwise gradient of hexane through mixtures of $CH_2Cl_2$:hexane to EtOAc: $CH_2Cl_2$ (30:70) to give the product as a pale yellow oil; TLC, $R_f = 0.21$, silica gel, $CH_2Cl_2$:petroleum ether (50:50).

d. L-Valinal diethylacetal (Formula XVIIIa, $R^1 = CH(CH_3)_2$)

A mixture of the product of Example 1c (147.8 g, 0.478 mol) and 10% Pd/C (10 g) in EtOAc (1500 ml) was stirred under $H_2$ (1 atm) until 2500 ml of $H_2$ were consumed. Twice during this time the reaction was interrupted and 10% Pd/C (10 g) was added. The reaction mixture was then filtered through a pad of diatomaceous earth. 10% Pd/C (10 g) was added and the reaction mixture stirred until 10.92 liters of $H_2$ were consumed. The reaction mixture was filtered through diatomaceous earth and the filtrate was concentrated under vacuum to give the product (78.8 g) as a pale yellow oil; $[alpha]_D^{25} = +7.8$.

e. (Formula XVb, $R^1 = CH(CH_3)_2$, $R^3A = \emptyset CH_2OCO$-)

WSCDI (24.04 g, 125.4 mmol) was added to a stirred solution of CBZ-L-proline (28.4 g, 114.1 mmol), HOBT (30.00 g, 228.2 mmol), the product of Example 1d (20 g, 114.1 mmol) and dry THF (400 ml) at 0°C under $N_2$. The resulting mixture was stirred at 0°C for 1 hr; the cooling bath was removed and the mixture was allowed to warm to room temperature and was stirred at room temperature overnight. The THF was removed under vacuum to afford crude product. The crude product was dissolved in ethyl acetate and the ethyl acetate solution was successively washed with 1N HCl, saturated $NaHCO_3$, and brine. The ethyl acetate solution was then dried ($MgSO_4$), filtered and the filtrate was concentrated under vacuum to afford, after purification by flash chromatography ($CHCl_3$:MeOH (98:2)), the product (35.03 g, 77%) as a yellow oil; TLC, $R_f = 0.7$, $CHCl_3$:MeOH (95:5).

f. (Formula VIb, $R^1 = CH(CH_3)_2$, $R^3A = \emptyset CH_2OCO$-)

A solution of the product from Example 1e (24.0 g, 60 mmol), p-toluenesulfonic acid (2.4 g, 12.6 mmol) and acetone (1600 ml) was stirred at room temperature for 5 hr. The acetone was removed under vacuum to leave an oily residue. This residue was dissolved in chloroform and the solution was washed with saturated $NaHCO_3$ and brine, dried ($MgSO_4$), filtered and the filtrate was concentrated under vacuum to give the product (17.67 g, 91%) as a light amber oil; TLC, $R_f = 0.46$, $CHCl_3$:$CH_3OH$ (95:5).

g. (Formula VIIb, $R^1 = CH(CH_3)_2$, $R^3A = \emptyset CH_2OCO$-)

A mixture of the product from Example 1f (2.50 g, 7.75 mmol), ethyl 2-bromo-2,2-difluoroacetate (1.57 g, 7.75 mmol), activated zinc (0.505 g, 7.75 mmol) and dry THF (125 ml) was heated at gentle reflux under nitrogen for 1 hr. The mixture was then allowed to cool to just below reflux and additional ethyl 2-bromo-2,2-difluoroacetate (1.57 g, 7.75 mmol) and activated zinc (0.505 g, 7.75 mmol) were added. The reaction mixture was again heated to gentle reflux and kept at reflux for 3 hr. The mixture was cooled and ethyl acetate (400 ml) was added. The ethyl acetate solution was washed with 1N $KHSO_4$ solution and brine, dried over $MgSO_4$, filtered and the filtrate was concentrated under vacuum to afford a crude product (3.7 g).

The product was purified by flash chromatography (EtOAc:hexane (1:1)) to give 1.53 g (45% yield) of the desired product as a light yellow waxy solid; TLC, $R_f = 0.45$, petroleum ether:EtOAc (33:66).

h. (Formula IXb, $R^1 = CH(CH_3)_2$, $R^3A = ØCH_2OCO$-)

1N Sodium hydroxide solution (2.75 ml, 2.75 mmol) was added to a stirred solution of the product from Example 1g (1.0 g, 2.19 mmol) and methanol (15 ml) at room temperature. The resulting solution was stirred at room temperature for 4 hr. The reaction mixture was treated with water (75 ml) and the resulting solution was extracted with EtOAc. The aqueous layer was made acidic (pH 2) with 1N HCl. The acidic aqueous mixture was extracted with ethyl acetate, and the ethyl acetate layer was washed with brine, dried ($MgSO_4$), filtered and the filtrate was concentrated under vacuum to give the product (0.883 g, 94.1% yield) as a white foam; TLC, $R_f = 0.1$, $CHCl_3$: MeOH:HOAc (95:5:0.5).

i. (Formula VIIIb, $R^1 = CH(CH_3)_2$, $R^3A = ØCH_2OCO$-, $R^B = CH_2$-$CH_2COOCH_2CH_3$, $R^C = H$)

WSCDI (0.251 g, 1.27 mmol) was added to a stirred solution of the product from Example 1h (0.5 g, 1.16 mmol), beta-alanine ethyl ester hydrochloride (0.179 g, 1.16 mmol), HOBT (0.158 g, 1.16 mmol), N-methylmorpholine (0.117 g, 1.16 mmol) and dry THF (20 ml) at ambient temperature under $N_2$. The resulting mixture was stirred at ambient temperature overnight. The mixture was concentrated under vacuum, and the resulting residue was dissolved in ethyl acetate. The ethyl acetate solution was washed with 1N HCl, saturated $NaHCO_3$ and brine, dried ($MgSO_4$), filtered and the filtrate was concentrated under vacuum to afford the product (61.6%); TLC, silica gel, $R_f = 0.4$, MeOH:$CHCl_3$ (5:95).

j. (Formula Ib, $R^1 = CH(CH_3)_2$, $R^3A = ØCH_2OCO$-, $X = NR^C$, $R^B = CH_2CH_2COOCH_2CH_3$, $R^C = H$)

To a solution of the product of Example 1i (0.5 g, 0.95 mmol) and dry $CH_2Cl_2$ (25 ml) was added Dess-Martin periodinane (2.01 g, 4.74 mmol). Trifluoroacetic acid (0.54 g, 4.74 mmol) was added and the solution was allowed to stir at ambient temperature under $N_2$ overnight. Ethyl acetate (75 ml) was added and the mixture was extracted with saturated $Na_2S_2O_3$, saturated $NaHCO_3$ and brine. The organic layer was dried ($MgSO_4$) and concentrated under vacuum to afford the product (42%) as an oil; TLC, silica gel, $R_f = 0.7$, MeOH:$CHCl_3$ (5:95); $^1$H NMR (DMSO-$d_6$): 0.79(m,6), 1.17(t,J = 7.5Hz,3), 1.80(m,3), 2.14(m,2), 2.5(m,2), 3.38-(m,4), 4.05(q,J = 7.5Hz,2), 4.36(m,1), 4.7(m,1), 4.9(m,2), 7.32(m,5), 8.35(m,1), 9.15(m,1).

Example 2

2-[[[3,3-Difluoro-1-(1-methylethyl)-2,4-dioxo-4-[[2-(2-pyridinyl)ethyl]amino]butyl]amino]carbonyl]-1-pyrrolidinecarboxylic acid phenylmethyl ester (Formula Ib, $R^A$ = Formula II, $R^1 = CH(CH_3)_2$, $R^3A = ØCH_2OCO$-, $X = NR^C$, $R^B = CH_2CH_2$(2-pyridyl), $R^C = H$)

a. (Formula XXVIb, $R^1 = CH(CH_3)_2$, $R^3A = ØCH_2OCO$-)

A solution of the product of Example 1g (1.3 g, 2.85 mmol) and $CH_2Cl_2$ (5.0 ml) was added to a stirred mixture of Dess-Martin periodinane (4.83 g, 11.39 mmol) and $CH_2Cl_2$ (35 ml). Trifluoroacetic acid (0.89 ml) was added and the reaction mixture was stirred at room temperature under $N_2$ overnight. Ethyl acetate (200 ml) was added and the mixture was extracted with saturated $Na_2S_2O_3$, saturated $NaHCO_3$ and brine. The organic layer was dried ($MgSO_4$), filtered and concentrated under vacuum to afford, after purification by flash chromatography (MeOH:$CHCl_3$ (1:99)), the product (0.53 g); TLC, $R_f = 0.65$, MeOH: $CHCl_3$ (2:98).

b. (Formula Ib, $R^A$ = Formula II, $R^1 = CH(CH_3)_2$, $R^3A = ØCH_2OCO$-, $X = NR^C$, $R^B = CH_2CH_2$(2-pyridyl), $R^C = H$)

A solution of product prepared by the method of Example 2a (0.460 g, 1.01 mmol), 2-(2-pyridyl)-ethylamine (0.12 ml, 1.01 mmol) and ethanol (1.5 ml) was stirred at room temperature under $N_2$ for 1 hour. The ethanol was removed under vacuum to give a residue which was purified by flash chromatography (MeOH:$CHCl_3$ (3:97)), to afford the product (0.135 g, 20%); TLC, $R_f = 0.68$, MeOH:$CHCl_3$ (5:95); $^1$H NMR (DMSO-$d_6$): 0.78(m,6), 1.80(m,3), 2.16(m,2), 2.92(t,J = 7.5Hz,2), 3.40(m,4); 4.4(m,1), 4.8(m,1), 4.95(m,2), 7.30(m,7), 7.6(m,1), 8.3(m,2), 9.2(m,1).

Example 3

2-[[[3,3-Difluoro-4-[(2-hydroxyethyl)amino]-1-(1-methylethyl)-2,4-dioxobutyl]amino]carbonyl]-1-pyrrolidinecarboxylic acid phenylmethyl ester (Formula Ib, $R^A$ = Formula II, $R^1 = CH(CH)_3)_2$, $R^3A$ = ∅$CH_2OCO$-, X = $NR^C$, $R^B = CH_2CH_2OH$, $R^C$ = H)

Using the method of Example 2b, product prepared by the method of Example 2a was allowed to react with 2-ethanolamine to afford, after purification by flash chromatography (MeOH:$CHCl_3$ (2:98)), the title product (83%); TLC, $R_f$ = 0.3, MeOH:$CHCl_3$ (2:98).

| Analysis calculated for | | | |
|---|---|---|---|
| $C_{22}H_{29}N_3O_6F_2.0.5H_2O$: | C, 55.22; | H, 6.31; | N, 8.78 |
| Found: | C, 55.03; | H, 6.13; | N, 8.63 |

Example 4

2-[[[3,3-Difluoro-4[(3-hydroxypropyl)amino]-1-(1-methylethyl)-2,4-dioxobutyl]amino]carbonyl]-1-pyrrolidinecarboxylic acid phenylmethyl ester (Formula Ib, $R^A$ = Formula II, $R^1 = CH(CH_3)_2$, $R^3A$ = ∅$CH_2OCO$-, X = $NR^C$, $R^B = (CH_2)_3OH$, $R^C$ = H)

Using the method of Example 2b, the product prepared by the method of Example 2a was allowed to react with 3-propanolamine to afford, after purification by flash chromatography (MeOH:$CHCl_3$ (3:97)), the title product (65%); $^1$NMR (DMSO-$d_6$): 0.8(m,6), 1.6(m,2), 1.8(m,3) 2.2(m,2), 3.2(m,2), 3.4(m,4), 4.36(m,1), 4.8(m,1), 5.0(m,2), 7.3(m,5), 8.34(m,1), 9.05(m,1).

Example 5

2-[[[3,3-Difluoro-4-[(2-hydroxy-2-phenylethyl)amino]-1-(1-methylethyl)-2,4-dioxobutyl]amino]carbonyl]-1-pyrrolidinecarboxylic acid phenylmethyl ester (Formula Ib, $R^A$ = Formula II, $R^1 = CH(CH_3)_2$, $R^3A$ = ∅$CH_2OCO$-, X = $NR^C$, $R^B = CH_2CH(OH)$∅, $R^C$ = H)

Using the method of Example 2b, product prepared by the method of Example 2a was allowed to react with 2-phenyl-2-ethanolamine to afford, after purification by flash chromatography (MeOH:$CHCl_3$ (2:98)), the product (42%); TLC, $R_f$ = 0.73, (MeOH:$CHCl_3$ (5:95)).

| Analysis calculated for | | | |
|---|---|---|---|
| $C_{28}H_{33}F_2N_3O_6$: | C, 61.64; | H, 6.09; | N, 7.70 |
| Found: | C, 61.26; | H, 6.14; | N, 7.60 |

Example 6

2-[[[3,3-Difluoro-1-(1-methylethyl)-2,4-dioxopentyl]-amino]carbonyl]-1-pyrrolidinecarboxylic acid phenylmethyl ester (Formula Ib, $R^A$ = Formula II, $R^1 = CH(CH_3)_2$, $R^3A$ = ∅$CH_2OCO$-, $XR^B = CH_3$)

a. (Formula XIIb, $R^1 = CH(CH_3)_2$, $R^3A$ = ∅$CH_2OCO$-)

1-(3-Dimethylaminopropyl)-3-ethylcarbodiimide hydrochloride (0.24 g, 1.23 mmol) was added to a suspension of the product of Example 1h (0.48 g, 1.12 mmol), O,N-dimethylhydroxylamine hydrochloride (0.11 g, 1.12 mmol), 1-hydroxybenzotriazole (0.3 g, 2.24 mmol) and N-methylmorpholine (0.11 g, 1.12 mmol) in $CH_2Cl_2$ (10 ml). The reaction was stirred at room temperature overnight and concentrated under vacuum. The residue was taken up in EtOAc, washed with saturated aqueous $NaHCO_3$, 1N aqueous HCl and brine, dried ($Na_2SO_4$), filtered and the filtrate concentrated under vacuum to give the product (0.42 g); TLC, silica gel, $R_f$ = 0.38, MeOH:$CHCl_3$ (5:95).

b. (Formula Xb, $R^1 = CH(CH_3)_2$, $R^3A = \emptyset CH_2OCO$-, $R^B = H$)

Methylmagnesium bromide ((2.9 M in $Et_2O$ (1.0 ml, 2.9 mmol)) was added to a cooled (0° C) solution of the product of Example 6a (0.355 g, 0.75 mmol) in THF (5 ml). The reaction was stirred for 1 hr at 0° C and 1N aqueous HCl was added. The mixture was extracted with EtOAc and the combined extracts were washed with brine, dried ($Na_2SO_4$), filtered and concentrated under vacuum to give the product (0.31 g); TLC, silica gel, $R_f = 0.40$, MeOH:$CHCl_3$ (5:95).

c. (Formula Ib, $R^1 = CH(CH_3)_2$, $R^3A = \emptyset CH_2OCO$-, $X = CH_2$, $R^B = H$)

A solution of the product of Example 6b (0.3 g, 0.7 mmol), Dess-Martin periodinane (0.59 g, 1.4 mmol) and TFA (0.055 ml, 0.7 mmol) in dry $CH_2Cl_2$ (5 ml) was stirred for 3 hr at room temperature. EtOAc was added and the mixture was washed with saturated aqueous $Na_2S_2O_3$, saturated aqueous $NaHCO_3$ and brine, dried ($Na_2SO_4$), filtered and concentrated to give a crude product. The product was purified by flash chromatography using EtOAc:hexane (2:3) as eluant to give the product (0.21 g); TLC, silica gel, $R_f = 0.36$, EtOAc:hexane, (50:50).

Example 7

2-[[[4-[(2-Carboxyethyl)amino]-3,3-difluoro-1-(1-methylethyl)-2,4-dioxobutyl]amino]carbonyl]-1-pyrrolidinecarboxylic acid phenylmethyl ester (Formula Ib, $R^A$ = Formula II, $R^1 = CH(CH_3)_2$, $R^3A = \emptyset CH_2OCO$-, $X = NR^C$, $R^B = CH_2CH_2COOH$, $R^C = H$)

1N NaOH (0.4 ml, 0.4 mmol) was added to a stirred solution of the product from Example 1j (0.17 g, 0.32 mmol), methanol (2.0 ml) and water (1.5 ml). The mixture was stirred at room temperature for 12 hr. Water (25 ml) was added and the aqueous mixture was extracted with ethyl acetate. The aqueous layer was made acidic (pH 2) with 1N NaOH solution. This acidic mixture was extracted (2 times) with ethyl acetate. The ethyl acetate solution was washed with 1N HCl solution and brine, dried ($MgSO_4$), filtered and the filtrate was concentrated under vacuum to give the title compound (0.128 g, 81%) as a dry white foam; TLC, silcia gel, $R_f = 0.48$, $CHCl_3$: $CH_3OH$:HOAc (95:5:0.5.)

| Analysis calculated for | | | |
|---|---|---|---|
| $C_{23}H_{29}F_2N_3O_7.\frac{1}{2}H_2O$: | C, 54.54; | H, 5.97; | N, 8.30 |
| Found: | C, 54.62; | H, 5.84; | N, 7.89 |

Example 8

2-[[[4-[[3-(4-Chlorophenyl)sulfonylamino-3-oxopropyl]-amino]-3,3-difluoro-1-(1-methylethyl)-2,4-dioxobutyl]-amino]carbonyl]-1-pyrrolidinecarboxylic acid phenylmethyl ester (Formula Ib, $R^A$ = Formula II, $R^1$ = CH-$(CH_3)_2$, $R^3A = \emptyset CH_2OCO$-, $X = NR^C$, $R^B = CH_2CH_2CONHS(O)_2(4\text{-}Cl\emptyset)$, $R^C = H$)

To a stirred solution of 4-chlorobenzenesulfonamide (0.01926 g, 0.10 mmol) in dry $CH_2Cl_2$ (2 ml) at ambient temperature under $N_2$ was added DMAP (0.01343 g, 0.11 mmol), followed by WSCDI (0.02108 g, 0.11 mmol) and material prepared as described in Example 7 (0.050 g, 0.10 mmol). After the reaction mixture had been stirred at ambient temperature overnight, additional $CH_2Cl_2$ (20 ml) was added and the solution was washed with 1N HCl and brine, dried ($MgSO_4$), filtered and concentrated under vacuum to give 60 mg of crude product which was purified by flash chromatography on acidic silica gel, eluting with $CHCl_3$:MeOH (99:1), to afford the title product (0.029 g, 43%) as a white powder; TLC, silica gel, $R_f = 0.46$, $CHCl_3$:MeOH:HOAc (98:2:0.1); HPLC, $t_R = 10.08$ min, $H_2O$:$CH_3CN$:THF:TFA (55:35:15:0.1), 2 ml/min, Phenomenex® Zorbax® C-8 analytical column, 4.6 mm x 35 cm; $^1H$ NMR (DMSO-$d_6$): 0.77(m,6), 1.79(m,3), 2.17(m,2), 4.3(m,1), 4.6-5.06(m,3), 7.31(m,5), 7.68(d,J = 8.675Hz, 2), 7.9(d,J = 8.65Hz,2), 8.35(m,1), 9.31-(m,1), 12.29 (m,1).

Example 10

2-[[[3,3-Difluoro-1-(1-methylethyl)-4-[methyl(2-phenylethyl)amino]-2,4-dioxobutyl]amino]carbonyl]-1-pyrrolidinecarboxylic acid phenylmethyl ester (Formula Ib, $R^3A = \emptyset CH_2OCO$-, $R^1 = -CH(CH_3)_2$, $R^A = -COXR^B$, $X = NR^C$, $R^C = CH_3$, $R^B = -(CH_2)_2\emptyset$)

a. (Formula VIIIb, $R^3A = \emptyset CH_2OCO$-, $R^1 = CH(CH_3)_2$, $R^B = -(CH_2)_2\emptyset$, $R^C = CH_3$)

Using the method of Example 1i, but eliminating the use of N-methylmorpholine, a product prepared by the method of Example 1h was allowed to react with N-methyl-2-phenethylamine to afford, without further purification, a product (68%); TLC, $R_f = 0.44$, silica gel, MeOH:$CHCl_3$ (5:95).

b. (Formula Ib, $R^3A = \emptyset CH_2OCO$-, $R^1 = -CH(CH_3)_2$, $R^A = -COXR^B$, $X = NR^C$, $R^C = CH_3$, $R^B = -(CH_2)_2\emptyset$)

Using the method of Example 1j, but extracting first with saturated $NaHCO_3$ containing 23 equivalents of $Na_2S_2O_3$, followed by saturated $NaHCO_3$ extraction, the product of Example 10a was oxidized to afford, after purification by flash chromatography ($CHCl_3$), the title product (37%); HPLC, $t_R = 26.46$ minutes, $CH_3CN$:$H_2O$ (50:50), 2 ml/min, Zorbax ODS analytical.

| Analysis calculated for | | | |
|---|---|---|---|
| $C_{29}H_{35}F_2N_3O_5.H_2O$: | C, 62.01; | H, 6.64; | N, 7.48 |
| Found: | C, 62.26; | H, 6.39; | N, 7.56 |

Example 11

2-[[[4-[(3-Carboxypropyl)amino]-3,3-difluoro-(1-methylethyl-2,4-dioxobutyl]amino]carbonyl]-1-pyrrolidinecarboxylic acid phenylmethyl ester (Formula Ib, $R^3A = \emptyset CH_2OCO$-, $R^1 = -CH(CH_3)_2$, $R^A = -COXR^B$, $X = NR^C$, $R^B = -(CH_2)_3COOH$, $R^C = H$)

a. (Formula VIIIb, $R^3A = \emptyset CH_2OCO$-, $R^1 = CH(CH_3)_2$, $R^B = -(CH_2)_3COOCH_3$, $R^C = H$)

Using the method of Example 1i, a product prepared by the method of Example 1h was allowed to react with $\gamma$-aminobutyric acid methyl ester hydrochloride to afford, without further purification, a product (93%); TLC, $R_f = 0.51$, silica gel, MeOH:$CHCl_3$ (5:95).

b. (Formula Ib, $R^3A = \emptyset CH_2OCO$-, $R^1 = CH(CH_3)2$, $R^A = COXR^B$,$X = NR^C$, $R^B = -(CH_2)_3COOCH_3$, $R^C = H$)

Using the method of Example 10b the product of Example 11a was oxidized to afford, after purification by flash chromatography (MeOH:$CHCl_3$ (3:97)), a product (83%); TLC, $R_f = 0.66$, silica gel, MeOH:$CHCl_3$ - (5:95).

c. (Formula Ib, $R^1 = -CH(CH_3)_2$, $R^A = -COXR^B$, $X = NR^C$, $R^B = (CH_2)_3COOH$, $R^C = H$)

Using the method of Example 1h, but using $H_2O$:MeOH in a 1:4 ratio, the product of Example 11b was hydrolyzed to afford, after purification by flash chromatography on acidic silica gel (MeOH:$CHCl_3$ (5:95)), the title product (77%); HPLC, $t_R = 21.22$ minutes, 2 ml/min, $H_2O$:$CH_3CN$:THF:TFA (55:35:15:0.1), $C_8$ column.

| Analysis calculated for | | | |
|---|---|---|---|
| $C_{29}H_{31}F_2N_3O_7.0.5\ H_2O$: | C, 55.37; | H, 6.17; | N, 7.81 |
| Found: | C, 55.32; | H, 6.03; | N, 7.81 |

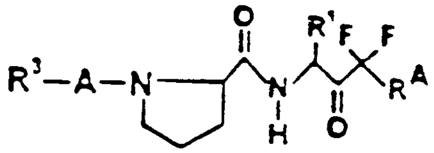

Ib

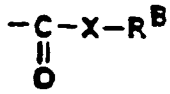

II

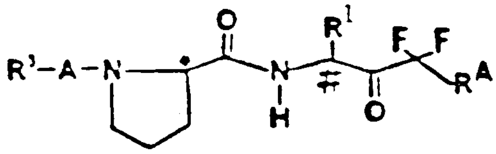

IIIb

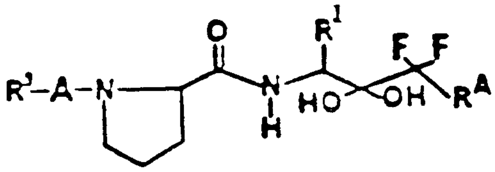

IVb

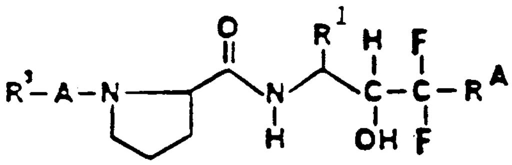

Vb

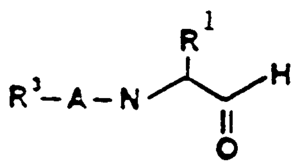

VIa

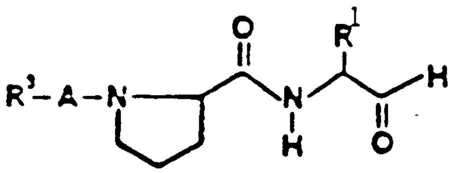

VIb

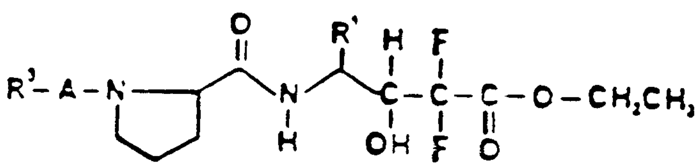

VIIb

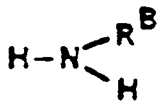

Vd

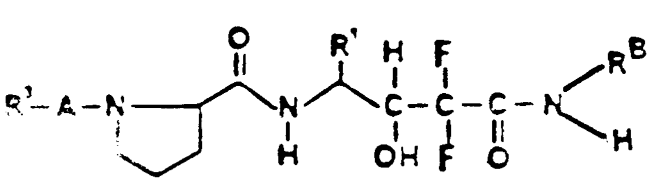

VIIIb

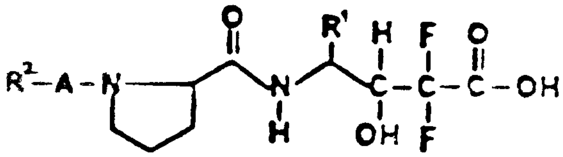

IXb

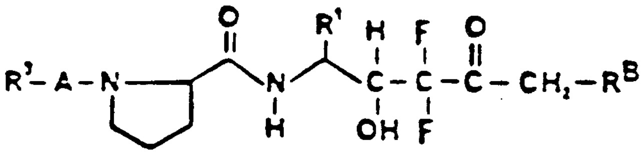

Xb

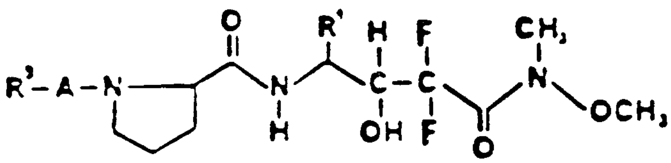

XIIb

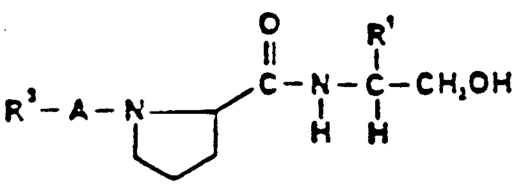

XIVb

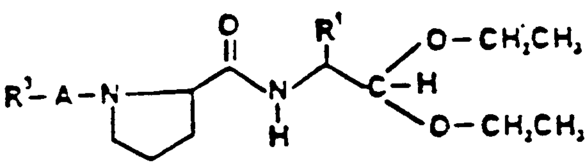

XVb

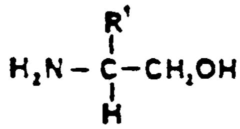

XVI

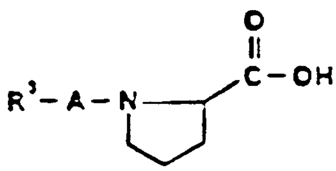

XVIIb

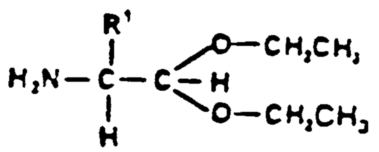

XVIIIa

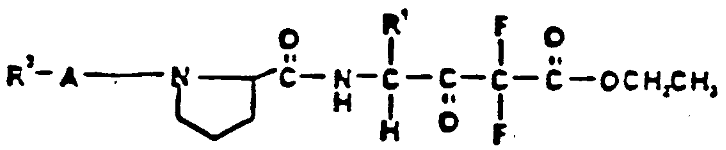

XXVIb

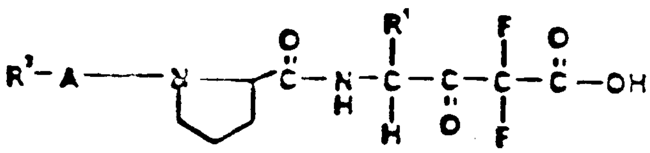

XXVIIb

**Claims**

**Claims for the following Contracting States : BE, CH, DE, FR, GB, IT, LI, NL**

**1.** A compound of the formula Ib:

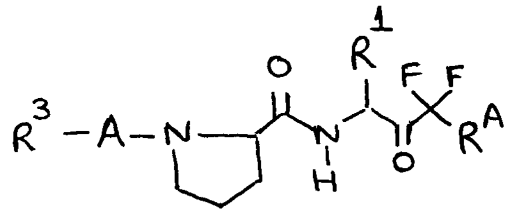

Ib

wherein $R^1$ is (1-3C) alkyl; $R^3$ is benzyl;
$R^A$ is a group of the formula -CO.X.$R^B$ wherein X.$R^B$ is methyl or X is the group -NH- and $R^B$ is selected from:- $-CH_2CH_2CO_2CH_2CH_3$, $-CH_2CH_2$(2-pyridyl), $-CH_2CH_2OH$, $-CH_2CH_2CH_2OH$, $-CH_2CH(OH)$(phenyl), $-CH_2CH_2CO_2H$ and $-CH_2CH_2CONHS(O)_2$(4-chlorophenyl); and A is oxycarbonyl; or a pharmaceutically acceptable salt thereof.

2. A compound as claimed in claim 1 wherein $R^1$ is isopropyl.

3. A compound as claimed in claim 1 or 2 wherein a chiral centre at $R^1$ has an S configuration.

4. A salt as claimed in any one preceding claim which is selected from alkali metal, alkali earth metal and organic amine salts, and from salts with acids forming pharmaceutically acceptable acid addition salts.

**Claim for the following Contracting State : ES**

1. A process for producing a compound of the formula Ib:

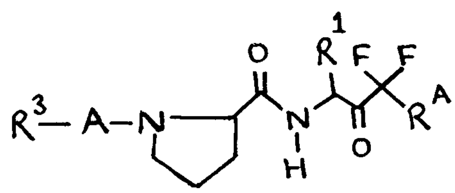

Ib

wherein $R^1$ is (1-3C) alkyl; $R^3$ is benzyl;
$R^A$ is a group of the formula -CO.X.$R^B$ wherein X.$R^B$ is methyl or X is the group -NH- and $R^B$ is selected from:- $-CH_2CH_2CO_2CH_2CH_3$, $-CH_2CH_2$(2-pyridyl), $-CH_2CH_2OH$, $-CH_2CH_2CH_2OH$, $-CH_2CH(OH)$(phenyl), $-CH_2CH_2CO_2H$ and $-CH_2CH_2CONHS(O)_2$(4-chlorophenyl); and A is oxycarbonyl; or a pharmaceutically acceptable salt thereof as appropriate; which is characterised by:-

(a) oxidising a compound of the formula Vb:

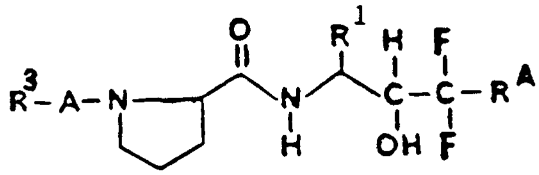

Vb

with an oxidising agent;

(b) reacting an ester of the formula XXVIb:

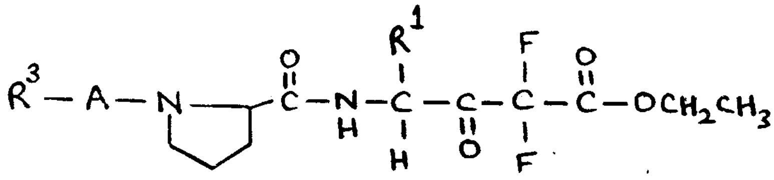

XXVIb

with an amine of the formula $R^B.NH_2$;
(c) coupling an acid of the formula XXVIIb:

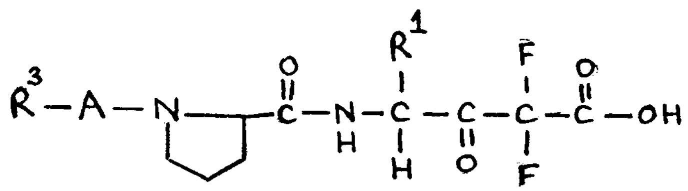

XXVIIb

with an amine of the formula $R^B.NH_2$;
(d) for compounds where $R^B$ contains a carboxy group, decomposing an ester of a corresponding compound of formula Ib; or
(e) for compounds where $R^B$ contains a (4-chlorophenyl)sulphonylaminocarbonyl group, reacting a corresponding compound of the formula Ib where $R^B$ contains a carboxy group with 4-chlorophenylsulphonamide in the presence of a dehydrating agent in a solvent or diluent;

whereafter, when a pharmaceutically aaceptable salt is required it may be obtained by reaction of said compound of formula Ib with the appropriate acid or base affording a physiologically acceptable anion or cation, respectively;
and wherein the variables have any of the meanings defined above unless otherwise stated.

**Patentansprüche**
**Patentansprüche für folgende Vertragsstaaten : BE, CH, DE, FR, GB, IT, LI, NL**

1. Verbindung der Formel Ib,

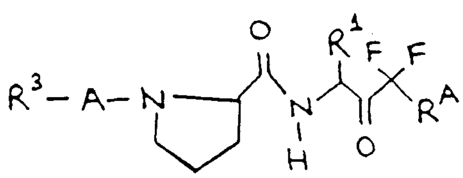

Ib

worin $R^1$ für (1-3C)Alkyl steht, $R^3$ für Benzyl steht, $R^A$ für eine Gruppe der Formel -CO.X.$R^B$ steht, worin X.$R^B$ für Methyl steht oder X für die Gruppe -NH- steht und $R^B$ ausgewählt ist aus $-CH_2CH_2CO_2CH_2CH_3$, $-CH_2CH_2$(2-Pyridyl), $-CH_2CH_2OH$, $-CH_2CH_2CH_2OH$, $-CH_2CH(OH)$(Phenyl), $-CH_2CH_2CO_2H$ und $-CH_2CH_2CONHS(O)_2$(4-Chlorophenyl), und A für Oxycarbonyl steht; oder ein pharmazeutisch zulässiges Salz davon.

2. Verbindung nach Anspruch 1, worin $R^1$ für Isopropyl steht.

3. Verbindung nach Anspruch 1 oder 2, worin ein chirales Zentrum bei $R^1$ die S-Konfiguration aufweist.

4. Salz nach einem der vorhergehenden Ansprüche, welches ausgewählt ist aus Alkalimetall-, Erdalkalimetall- und organischen Amin-Salzen und aus Salzen mit Säuren, die pharmazeutisch zulässi-

ge Säureadditionssalze bilden.

**Patentanspruch für folgenden Vertragsstaat : ES**

1. Verfahren zur Herstellung einer Verbindung der Formel Ib,

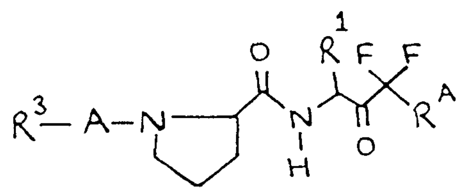

Ib

worin $R^1$ für (1-3C)Alkyl steht, $R^3$ für Benzyl steht, $R^A$ für eine Gruppe der Formel -CO.X.$R^B$ steht, worin X.$R^B$ für Methyl steht oder X für die Gruppe -NH- steht und $R^B$ ausgewählt ist aus $-CH_2CH_2CO_2CH_2CH_3$, $-CH_2CH_2$(2-Pyridyl), $-CH_2CH_2OH$, $-CH_2CH_2CH_2OH$, $-CH_2CH(OH)$(Phenyl), $-CH_2CH_2CO_2H$ und $-CH_2CH_2CONHS(O)_2$(4-Chlorophenyl), und A für Oxycarbonyl steht; oder eines geeigneten pharmazeutisch zulässigen Salzes davon, gekennzeichnet durch

a) Oxidation einer Verbindung der Formel Vb

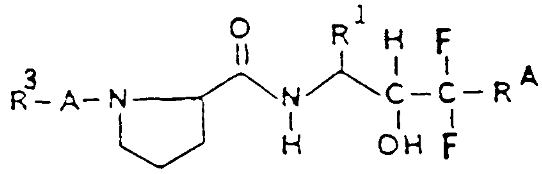

Vb

mit einem Oxidationsmittel,

b) Umsetzung eines Esters der Formel XXVIb

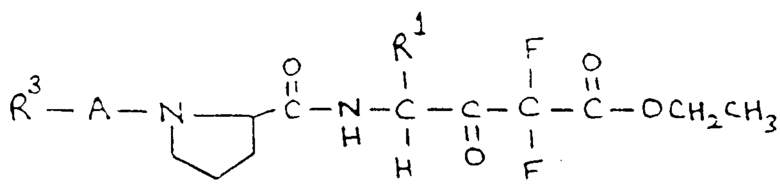

XXVIb

mit einem Amin der Formel $R^B.NH_2$;

c) Kupplung einer Säure der Formel XXVIIb

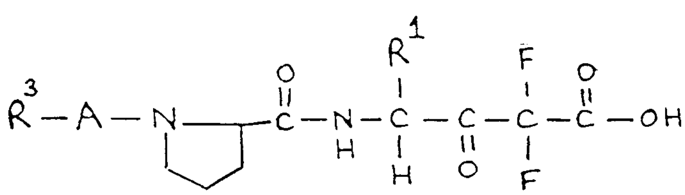

XXVIIb

mit einem Amin der Formel $R^B.NH_2$;

d) zur Herstellung solcher Verbindungen, worin $R^B$ eine Carboxy-Gruppe enthält, Zersetzung eines Esters einer entsprechenden Verbindung der Formel Ib; oder

e) zur Herstellung solcher Verbindungen, worin $R^B$ eine (4-Chlorophenyl)sulfonyl-aminocarbonyl-Gruppe enthält, Umsetzung einer entsprechenden Verbindung der Formel Ib, worin $R^B$ eine

Carboxy-Gruppe enthält, mit 4-Chlorophenylsulfonamid in Gegenwart eines Dehydratisierungsmittels in einem Lösungsmittel oder Verdünnungsmittel;

worauf, wenn ein pharmazeutisch zulässiges Salz gewünscht wird, dieses durch Umsetzung der Verbindung der Formel Ib mit der entsprechenden Säure oder Base, die ein physiologisch zulässiges Anion bzw. Kation liefert, erhalten wird;

wobei die Variablen die oben angegebenen Bedeutungen besitzen, sofern nichts anderes angegeben ist.

## Revendications

## Revendications pour les Etats contractants suivants : BE, CH, DE, FR, GB, IT, LI, NL

**1.** Composé de formule Ib :

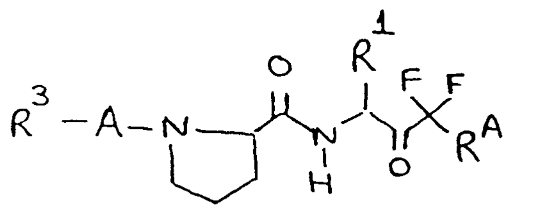

Ib

dans laquelle $R^1$ représente un groupe alkyle en $C_1$ à $C_3$ ; $R^3$ représente un groupe benzyle ; $R^A$ représente un groupe de formule $-CO.X.R^B$ dans laquelle $X.R^B$ représente un groupe méthyle ou bien X représente un groupe -NH- et $R^B$ est choisi entre les groupes $-CH_2CH_2CO_2CH_2CH_3$, $-CH_2CH_2$(2-pyridyle), $-CH_2CH_2OH$, $-CH_2CH_2CH_2OH$, $-CH_2CH(OH)$(phényle), $-CH_2CH_2CO_2H$ et $-CH_2CH_2CONHS(O)_2$(4-chlorophényle) ; et A représente un groupe oxycarbonyle ; ou un de ses sels pharmaceutiquement acceptables.

**2.** Composé suivant la revendication 1, dans lequel $R^1$ représente un groupe isopropyle.

**3.** Composé suivant la revendication 1 ou 2, dans lequel un centre chiral en $R^1$ possède une configuration S.

**4.** Sel suivant l'une quelconque des revendications précédentes, qui est choisi entre des sels de métaux alcalins, des sels de métaux alcalino-terreux et des sels d'amines organiques, et entre des sels formés avec des acides donnant des sels d'addition d'acides pharmaceutiquement acceptables.

## Revendication pour l'Etat contractant suivant : ES

**1.** Procédé de production d'un composé de formule Ib :

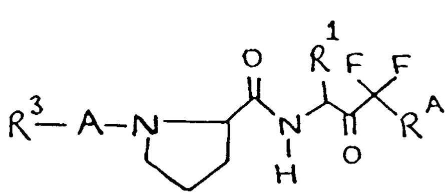

Ib

dans laquelle $R^1$ représente un groupe alkyle en $C_1$ à $C_3$ ; $R^3$ représente un groupe benzyle ; $R^A$ représente un groupe de formule $-CO.X.R^B$ dans laquelle $X.R^B$ représente un groupe méthyle ou bien X représente un groupe -NH- et $R^B$ est choisi entre les groupes $-CH_2CH_2CO_2CH_2CH_3$, $-CH_2CH_2$(2-pyridyle), $-CH_2CH_2OH$, $-CH_2CH_2CH_2OH$, $-CH_2CH(OH)$(phényle), $-CH_2CH_2CO_2H$ et $-CH_2CH_2CONHS(O)_2$(4-chlorophényle) ; et A représente un groupe oxycarbonyle ; ou d'un de ses sels pharmaceutiquement acceptables, de la manière appropriée ; qui est caractérisé en ce qu'il consiste :

(a) à oxyder un composé de formule Vb :

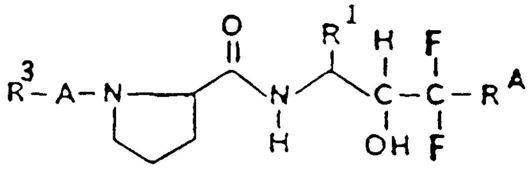

Vb

avec un agent oxydant ;
(b) à faire réagir un ester de formule XXVIb :

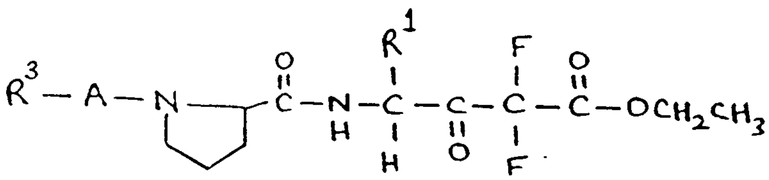

XXVIb

avec une amine de formule $R^B.NH_2$ ;
(c) à coupler un acide de formule XXVIIb :

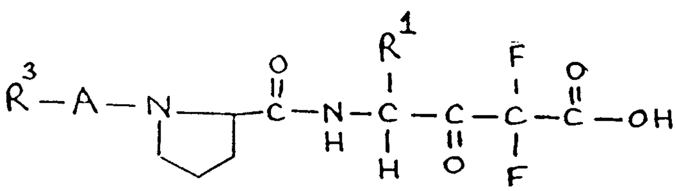

XXVIIb

avec une amine de formule $R^B.NH_2$ ;
(d) pour des composés dans lesquels $R^B$ contient un groupe carboxy, à décomposer un ester d'un composé correspondant de formule Ib ; ou
(e) pour des composés dans lesquels $R^B$ contient un groupe (4-chlorophényl)-sulfonylaminocarbonyle, à faire réagir un composé correspondant de formule Ib dans laquelle $R^B$ contient un groupe carboxy avec le 4-chlorophénylsulfonamide en présence d'un agent déshydratant dans un solvant ou diluent ;

puis, lorsqu'un sel pharmaceutiquement acceptable est requis, à faire réagir ledit composé de formule Ib avec l'acide ou la base appropriée donnant, respectivement, un anion ou cation physiologiquement acceptable, ce qui permet d'obtenir ce sel ;

les variables ayant n'importe lesquelles des définitions mentionnées ci-dessus, sauf spécification contraire.